# EUROPÄISCHE PATENTSCHRIFT

(11) **EP 0 505 723 B1**
(45) Veröffentlichungstag und Bekanntmachung des Hinweises auf die Patenterteilung: **02.08.1995**
(21) Anmeldenummer: 92102726.4
(22) Anmeldetag: 19.02.1992
(51) Int. Cl.: C07C 7/08, C10G 7/08

(54) **Verfahren zur Abtrennung von Aromaten durch Extraktivdestillation**
Process for the separation of aromatics by extractive distillation
Procédé pour la séparation des arômatiques par distillation extractive

(30) Priorität: 23.03.1991 DE 4109632
(43) Veröffentlichungstag der Anmeldung: 30.09.1992
(73) Patentinhaber: Krupp Koppers GmbH, 45143 Essen (DE)
(72) Erfinder: Skatulla, Luzian, W-4330 Mülheim/Ruhr (DE); Vollmer, Hans-Jürgen, Dr., W-4300 Essen (DE); Schneider, Hans-Christoph, W-4320 Hattingen (DE)

(56) Entgegenhaltungen:
- EP-A- 0 046 876
- DE-A- 2 263 344
- DE-A- 2 648 638
- FR-A- 1 470 472
- EIGTH WORLD PETROLEUM CONGRESS, PROCEEDINGS, Bd.4, 1971, LONDON, GB Seiten 213 - 219 E. MUELLER ET AL. 'Aromatics recovery by extractive distillation with N-methylpyrrolidone'
- VT "VERFAHRENSTECHNIK", Bd.14, Nr.9, 1980 Seiten 551 - 555 E. MÜLLER 'Gewinnung von Aromaten durch Extraktiv-Destillation'
- VERFAHRENSTECHNIK, Bd.8, Nr.3, 1974 Seiten 88 - 93 E. MÜLLER 'Gewinnung und Abtrennung von Aromaten durch Extraktion und Extraktivdestillation'

## Beschreibung

Dokument EP-A-0 046 876 offenbart ein Verfahren zur Aufarbeitung des Sumpfproduktes von Extraktivdestillationsprozessen zur Gewinnung reiner Kohlenwasserstoffe.

Die Erfindung betrifft ein Verfahren zur Abtrennung von Aromaten aus Kohlenwasserstoffgemischen beliebigen Aromatengehaltes durch Extraktivdestillation mit einem selektiven Lösungsmittel, bei dem die nichtaromatischen Bestandteile des als Einsatzprodukt dienenden Kohlenwasserstoffgemisches über Kopf aus der Extraktivdestillationskolonne abdestilliert werden, während die Aromaten zusammen mit dem verwendeten Lösungsmittel aus dem Sumpf der Extraktivdestillationskolonne abgezogen und in einer nachgeschalteten Abtreiberkolonne destillativ vom Lösungsmittel abgetrennt werden, wobei die Aromaten als Kopfprodukt und das Lösungsmittel als Sumpfprodukt aus der Abtreiberkolonne abgezogen werden, worauf das Lösungsmittel in die Extraktivdestillationskolonne wiedereingeleitet wird.

Bei der Anwendung des vorstehend genannten Verfahrens ist in der Praxis die Einhaltung von zwei Bedingungen unerläßlich. Einerseits darf der Nichtaromatengehalt in den gewonnenen Aromaten einen vorgegebenen Höchstwert nicht überschreiten und andererseits sollen die Aromatenverluste möglichst klein und die Ausbeute damit möglichst hoch sein. Die Erfüllung dieser Bedingungen ist jedoch mit einem bestimmten Energieaufwand verbunden, wobei für derartige Extraktivdestillationsverfahren üblicherweise Mittel- und Hochdruckdampf als Energieträger eingesetzt werden. Der Mitteldruckdampf dient dabei in erster Linie zur Beheizung der Extraktivdestillationskolonne, während der wesentlich teurere Hochdruckdampf in der Abtreiberkolonne für den Abtrieb der Aromaten vom Lösungsmittel benötigt wird.

Auf Grund der vorstehend skizzierten Gegebenheiten kann bei der Durchführung eines Verfahrens der eingangs genannten Art mit einer Einsparung bei den Energiekosten gerechnet werden, wenn für die Deckung des Energiebedarfs des Gesamtverfahrens ein Teil des bisher benötigten Hochdruckdampfes durch den billigeren Mitteldruckdampf ersetzt werden kann. Der Erfindung liegt deshalb die Aufgabe zugrunde, dieses Ziel zu verwirklichen.

Dies wird bei einem Verfahren der eingangs genannten Art erfindungsgemäß dadurch erreicht, daß nur ein Teil des Lösungsmittels mit der für den vollständigen Aromatenabtrieb erforderlichen hohen Temperatur aus dem Sumpf der Abtreiberkolonne abgezogen wird, während der Rest des Lösungsmittels mit einem gewissen Aromatengehalt und niedrigerer Temperatur als Seitenstrom aus der Abtreiberkolonne abgezogen wird, wobei dieser Seitenstrom an einer Stelle in die mit Rückfluß betriebene Extraktivdestillationskolonne wiedereingeleitet wird, die 6 bis 10 Böden unterhalb der Eintrittsstelle für den vom Sumpf der Abtreiberkolonne kommenden Lösungsmittelstrom liegt.

Das heißt, bei der Durchführung des erfindungsgemäßen Verfahrens bilden sich zwei Lösungsmittelkreisläufe mit unterschiedlicher Temperatur und unterschiedlichem Aromatengehalt aus. Die Erfindung geht dabei von der Erkenntnis aus, daß beim Abzug der gesamten Lösungsmittelmenge mit hoher Temperatur aus dem Sumpf der Abtreiberkolonne in der Praxis nur ein Teil des Wärmeinhaltes des abgezogenen Lösungsmittels durch Wärmeaustauschvorgänge innerhalb des Gesamtverfahrens genutzt werden kann. Ein Teil des Wärmeinhaltes muß jedoch stets vor der Wiedereinleitung des Lösungsmittels in die Extraktivdestillationskolonne durch Kühlung ungenutzt an die Umgebung abgeführt werden. Wenn nun, wie erfindungsgemäß vorgesehn, nur ein Teil des Lösungsmittels mit hoher Temperatur aus dem Sumpf der Abtreiberkolonne abgezogen wird, so verringert sich natürlich auch der Anteil der Restwärme entsprechend, der ungenutzt an die Umgebung abgeführt werden muß. Gleichzeitig verringert sich dadurch auch der für die Beheizung der Abtreiberkolonne erforderliche Hochdruckdampfbedarf.

Bei der Durchführung des erfindungsgemäßen Verfahrens hängen die Temperatur und der Aromatengehalt des als Seitenstrom aus der Abtreiberkolonne abgezogenen Lösungsmittels natürlich von der Lage der Abzugsstelle ab, wobei die Temperatur umso niedriger und der Aromatengehalt umso höher ist, je höher die Abzugsstelle an der Abtreiberkolonne angeordnet ist. Die Temperatur ihrerseits ist wiederum abhängig von der Siedetemperatur der zu gewinnenden Aromaten sowie der Siedetemperatur des verwendeten Lösungsmittels. So liegen beispielsweise bei der Gewinnung von Benzol durch Extraktivdestillation mit N-Formylmorpholin als Lösungsmittel die Temperatur des Lösungsmittelseitenstromes bei 149°C und der Benzolgehalt bei 2,78 Gew.-%, wenn der Abzug des Seitenstromes vom 29. Boden von oben aus der Abtreiberkolonne erfolgt. Wird der Abzug aus der Abtreiberkolonne dagegen vom 22. Boden von oben vorgenommen, so liegt die Temperatur des Seitenstromes bei 134,4°C und der Benzolgehalt bei 3,67 Gew.-%.

Die Aufteilung der beiden Lösungsmittelkreislaufströme erfolgt zweckmäßigerweise so, daß 55 bis 60 Gew.-% der Gesamtlösungsmittelmenge aus dem Sumpf und der Rest als Seitenstrom aus der Abtreiberkolonne abgezogen werden. Während der aus dem Sumpf der Abtreiberkolonne abgezogene Lösungsmittelteilstrom vor seiner Wiedereinleitung in die Extraktivdestillationskolonne in an sich bekannter Weise gekühlt wird, ist bei dem als Seitenstrom aus der Abtreiberkolonne abgezogenen Lösungsmittelteilstrom eine derartige Kühlung nicht vorgesehen. Dies bedeutet jedoch, daß mit diesem zweiten Teilstrom eine zusätzliche Wärmemenge der Extraktivdestillationskolonne in ihrem oberen Teil zugeführt wird. Deshalb ist es bei Anwendung des erfindungsgemäßen Verfahrens im Hinblick auf ein gutes Trennergebnis zweckmäßig, die Extraktivdestillationskolonne im Gegensatz zu der bisher vielfach üblichen Praxis mit äußerem Rückfluß zu betreiben. Um den Wärmeinhalt dieses zweiten Lösungsmittelteilstromes zu nutzen, ist gemäß einer bevorzugten Ausführungsform des erfindungsgemäßen Verfahrens außerdem vorgesehen, daß die Extraktivdestillationskolonne oberhalb der Lösungsmittelaufgabe einen zusätzlichen Kolonnenteil für die Abtrennung der Lösungsmittelreste von den Nichtaromaten aufweist.

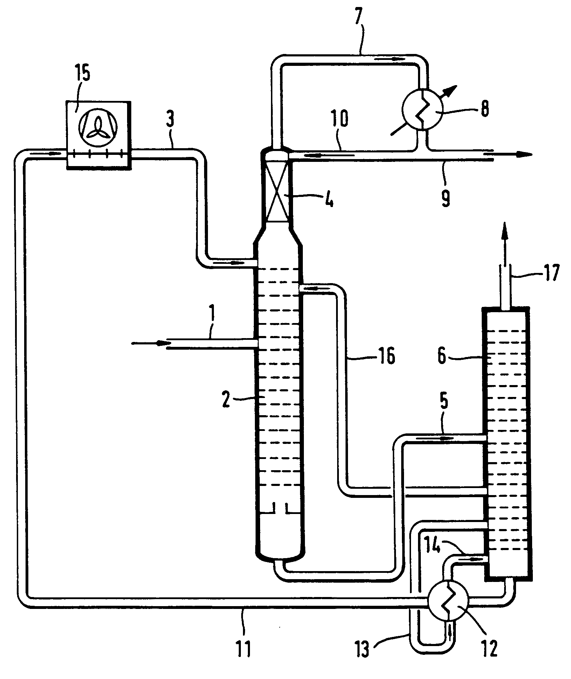

Weitere Einzelheiten des erfindungsgemäßen Verfahrens sollen an Hand des in der Abbildung dargestellten Fließschemas erläutert werden. Das Fließschema enthält dabei nur die für die Verfahrenserläuterung unbedingt notwendigen Anlagenteile, während Nebeneinrichtungen, wie beispielsweise Pumpen, Umlaufkocher, Wärmetauscher, Meß- und Regeleinrichtungen etc., nicht dargestellt sind.

Bei der Durchführung des erfindungsgemäßen Verfahrens wird das als Einsatzprodukt dienende aromatenhaltige Kohlenwasserstoffgemisch über die Leitung 1 in den mittleren Teil der mit Böden versehenen Extraktivdestillationskolonne 2 eingeleitet. Das erforderliche selektive Lösungsmittel wird über die Leitung 3 auf den Kopf der Extraktivdestillationskolonne 2 aufgegeben. Oberhalb der Lösungsmittelaufgabe weist die Extraktivdestillationskolonne 2 in diesem Falle einen zusätzlichen Kolonnenteil 4 auf, der der Abtrennung der Lösungsmittelreste von den Nichtaromaten dient. In der Extraktivdestillationskolonne 2 erfolgt die Auftrennung des Einsatzproduktes unter dem Einfluß des Lösungsmittels in an sich bekannter Weise. Das heißt, das eingeleitete Lösungsmittel fließt über die Böden dieser Kolonne nach unten herab, wobei es die dampfförmigen Aromaten aufnimmt. Das flüssige Sumpfprodukt, das aus dem Lösungsmittel und den darin gelösten Aromaten besteht, wird über die Leitung 5 aus der Extraktivdestillationskolonne 2 abgezogen und in die Abtreiberkolonne 6 eingeleitet, in der dieses vielfach auch als Extrakt bezeichnete Sumpfprodukt in seine Bestandteile zerlegt wird. Die nichtaromatischen Kohlenwasserstoffe des Einsatzkohlenwasserstoffgemisches, die die Raffinatphase bilden, steigen währenddessen in der Extraktivdestillationskolonne 2 dampfförmig nach oben. Damit aus diesen nichtaromatischen Kohlenwasserstoffen die Lösungsmittelreste entfernt werden können, weist die Extraktivdestillationskolonne 2 oberhalb der Lösungsmittelaufgabe, d.h. oberhalb der Einmündung der Leitung 3 in diese Kolonne, einen zusätzlichen Kolonnenteil 4 auf. Dieser Kolonnenteil 4, der mit Böden oder sonstigen Einbauten versehen sein kann, bildet zusammen mit der Extraktivdestillationskolonne 2 eine bauliche Einheit. In der vorliegenden Abbildung weist der Kolonnenteil 4 einen etwas geringeren Durchmesser auf als die übrige Extraktivdestillationskolonne 2. In der Praxis können aber beide Teile auch den gleichen Durchmesser besitzen. Die von den Lösungsmittelresten befreiten nichtaromatischen Kohlenwasserstoffe entweichen dampfförmig über Kopf aus dem Kolonnenteil 4 und gelangen über die Leitung 7 in den Kühler 8, in dem die Kohlenwasserstoffe kondensiert werden. Die Hauptmenge der flüssigen Nichtaromaten wird anschließend über die Leitung 9 aus dem Verfahren abgezogen und seiner weiteren Verwendung zugeführt, während ein Teilstrom über die Leitung 10 als Rückfluß am Kopf in den Kolonnenteil 4 wiedereingeleitet wird. Die Rückflußmenge wird dabei so eingestellt, daß die gewonnenen Nichtaromaten den gewünschten Reinheitsgrad aufweisen.

Erfindungsgemäß wird aus dem Sumpf der mit Böden versehenen Abtreiberkolonne 6 nur ein Teilstrom des Lösungsmittels mit der für den vollständigen Aromatenabtrieb erforderlichen hohen Temperatur über die Leitung 11 abgezogen. Wie bereits weiter oben feststellt wurde, kann ein Teil des Wärmeinhaltes dieses Teilstromes durch Wärmeaustauschvorgänge innerhalb des Gesamtverfahrens genutzt werden. Als Beispiel hierfür ist im vorliegenden Fließschema der Sumpfumlaufkocher 12 vorgesehen, der im Verlauf der Leitung 11 angeordnet ist, und der der Erhitzung des Sumpfproduktes aus der Abtreiberkolonne 6 dient. Über die Leitung 13 erfolgt dabei die Zufuhr des Sumpfproduktes zum Sumpfumlaufkocher 12 und über die Leitung 14 der Abzug desselben. Grundsätzlich sind natürlich innerhalb des Gesamtverfahrens auch noch andere Wärmeaustauschvorgänge zur Nutzung des Wärmeinhaltes des heißen Lösungsmittels möglich. Da dies jedoch nicht Gegenstand der vorliegenden Erfindung ist, braucht hierauf nicht näher eingegangen zu werden. Es ist aber immer so, daß auf diese Weise nur ein Teil des Wärmeinhaltes genutzt werden kann. Die noch vorhandene Restwärme muß dadurch ungenutzt abgeführt werden, daß das Lösungsmittel aus der Leitung 11 in den Luftkühler 15 eingeleitet wird, in dem es bis auf die Temperatur abgekühlt wird, mit der es über die Leitung 3 in die Extraktivdestillationskolonne 2 wiedereingeleitet werden kann. Da erfindungsgemäß nur zwischen 55 und 60 Gew.-% der Gesamtlösungsmittelmenge über die Leitung 11 aus der Abtreiberkolonne 6 abgezogen werden, verringert sich natürlich die Wärmenenge, die ungenutzt über den Luftkühler 15 abgeführt werden muß, entsprechend.

Das restliche Lösungsmittel wird über die Leitung 16 als Seitenstrom aus der Abtreiberkolonne 6 abgezogen und 6 bis 10 Böden unterhalb der Eintrittsstelle der Leitung 3 in die Extraktivdestillationskolonne 2 eingeleitet. Bei einer Abtreiberkolonne mit insgesamt 75 Böden kann die Abzugsstelle für diesen Seitenstrom beispielsweise zwischen dem 16. und 24. Boden von oben liegen. Wie bereits festgestellt wurde, ist hierbei die Temperatur des Seitenstromes umso niedriger und der Aromatengehalt umso höher, je höher die Abzugsstelle an der Abtreiberkolonne 6 angeordnet ist. Da der über die Leitung 16 abgezogene Lösungsmittelteilstrom vor seiner Wiedereinleitung in die Extraktivdestillationskolonne 2 keine Abkühlung erfährt, ist gemäß einer bevorzugten Ausführungsform des erfindungsgemäßen Verfahrens vorgesehen, die Extraktivdestillationskolonne 2 mit dem bereits weiter oben beschriebenen zusätzlichen Kolonnenteil 4 auszurüsten, in dem der Wärmeinhalt des über die Leitung 16 zugeführten Lösungsmittelteilstromes für die Entfernung der Lösungsmittelreste aus dem Raffinat genutzt wird. Die vom Lösungsmittel befreiten Aromaten werden über die Leitung 17 aus der Abtreiberkolonne 6 abgezogen.

Die Wirksamkeit des erfindungsgemäßen Verfahrens wird durch die nachfolgenden Versuchsergebnisse belegt. Hierbei ging es um die Abtrennung von Benzol durch Extraktivdestillation mit N-Formylmorpholin als selektivem Lösungsmittel. Als Einsatzprodukt diente dabei eine Rohbenzolfraktion. Im ersten Teil dieser Versuchsreihe wurde die Extraktivdestillation unter den bisher üblichen Bedingungen durchgeführt, wobei die Extraktivdestillationskolonne mit Mitteldruckdampf und die Abtreiberkolonne mit Hochdruckdampf beheizt wurde. Das heißt, das von den Aromaten befreite Lösungsmittel wurde in seiner Gesamtheit aus dem Sumpf der Abtreiberkolonne abgezogen und nach entsprechender Abkühlung in die ohne Rückfluß betriebene Extraktivdestillationskolonne zurückgeführt. Hierbei lag der Nichtaromatengehalt gewonnenen Benzols bei 170 ppm und die Benzolausbeute betrug 99,1 %.

Im zweiten Teil dieser Versuchsreihe wurde bei sonst unveränderten Verfahrensbedingungen erfindungsgemäß mit zwei Lösungsmittelkreisläufen gearbeitet. Dabei wurden nur ca. 57 Gew.-% des Lösungsmittels über die Leitung 11 aus der Abtreiberkolonne 6 abgezogen. Für das restliche Lösungsmittel erfolgte der Abzug als Seitenstrom über die Leitung 16. Hierbei erfolgte zunächst ein Abzug dieses Seitenstromes vom 29. Boden von oben der insgesamt mit 31 Böden versehenen Abtreiberkolonne 6. Die Temperatur dieses Seitenstromes lag dabei bei 149°C und der Benzolgehalt bei 2,78 Gew.-%. Der Seitenstrom wurde 7 Böden unterhalb des Eintritts der Leitung 3 in die Extraktivdestillationskolonne 2 eingeleitet. Bei einem Rückflußverhältnis in der Extraktivdestillationskolonne 2 von 2,3 wurde in diesem Falle der Vergleichswert von 170 ppm Nichtaromaten im gewonnenen Benzol erreicht. Bei einem Rückflußverhältnis von 2,0 wurde der Vergleichswert sogar unterschritten.

In einem weiteren Versuch wurde der Lösungsmittelseitenstrom vom 22. Boden von oben aus der Abtreiberkolonne 6 abgezogen und 7 Böden unterhalb des Eintritts der Leitung 3 in die Extraktivdestillationskolonne 2 eingeleitet. Die Temperatur des Seitenstromes lag in diesem Falle bei 134,3°C und der Benzolgehalt bei 3,67 Gew.-%. Der Vergleichswert von 170 ppm Nichtaromaten im gewonnenen Benzol wurde hierbei schon bei einem Rückflußverhältnis in der Extraktivdestillation von 1,8 erreicht.

Die vorliegenden Versuchsergebnisse bestätigen, daß die erfindungsgemäße Extraktivdestillation mit zwei Lösungsmittelkreisläufen durchführbar ist und zumindest zu gleich guten Ergebnissen führt wie die bisher übliche Arbeitsweise. Die durchgeführten Berechnungen haben dabei ergeben, daß der Energiebedarf für das Gesamtverfahren bei Anwendung der erfindungsgemäßen Arbeitsweise nicht wesentlich geringer ist als bei der Arbeitsweise nach dem Stand der Technik. Bei Anwendung der erfindungsgemäßen Arbeitsweise erfolgt jedoch eine Verschiebung der Wärmezufuhr von der Abtreiberkolonne 6 zur Extraktivdestillationskolonne 2, was eine Verringerung des Hochdruckdampfbedarfs bei gleichzeitiger Erhöhung des Mitteldruckdampfbedarfs bewirkt. So lag beim Abzug des Lösungsmittelseitenstromes vom 29. Boden der Abtreiberkolonne 6 der Hochdruckdampfbedarf nur noch bei ca. 75 % des Wertes für den im ersten Teil der Versuchsreihe durchgeführten Vergleichsversuch. Beim Abzug des Lösungsmittelseitenstromes vom 22. Boden der Abtreiberkolonne 6 konnte dieser Wert sogar auf ca.55 % des Wertes für den Vergleichsversuch gesenkt werden. Wegen der bestehenden Kostenrelation zwischen Hochdruck- und Mitteldruckdampf führt dies zu einer deutlichen Einsparung an Betriebskosten.

Das vorliegende Ergebnis findet seine Erklärung zum einen in der Tatsache, daß bei der erfindungsgemäßen Arbeitsweise nur ein Teil des Lösungsmittels auf die für den vollständigen Aromatenabtrieb erforderliche hohe Temperatur erhitzt wird. Zum anderen ist zu berücksichtigen, daß in diesem Falle im Sumpf der Abtreiberkolonne auch erheblich weniger Lösungsmittel verdampft werden muß, das für einen internen Rückfluß im unteren Teil der Abtreiberkolonne benötigt wird. Je nedriger der Aromatengehalt im Lösungsmittel sein soll, desto mehr Lösungsmittel muß dabei verdampft werden, was natürlich ebenfalls den Hochdruckdampfbedarf in die Höhe treibt.

## Patentansprüche

1. Verfahren zur Abtrennung von Aromaten aus Kohlenwasserstoffgemischen beliebigen Aromatengehaltes durch Extraktivdestillation mit einem selektiven Lösungsmittel, bei dem die nichtaromatischen Bestandteile des als Einsatzprodukt dienenden Kohlenwasserstoffgemisches über Kopf aus der Extraktivdestillationskolonne abdestilliert werden, während die Aromaten zusammen mit dem verwendeten Lösungsmittel aus dem Sumpf der Extraktivdestillationskolonne abgezogen und in einer nachgeschalteten Abtreiberkolonne destillativ vom Lösungsmittel abgetrennt werden, wobei die Aromaten als Kopfprodukt und das Lösungsmittel als Sumpfprodukt aus der Abtreiberkolonne abgezogen werden, worauf das Lösungsmittel in die Extraktivdestillationskolonne wiedereingeleitet wird, **dadurch gekennzeichnet,** daß nur ein Teil des Lösungsmittels mit der für den vollständigen Aromatenabtrieb erforderlichen hohen Temperatur aus dem Sumpf der Abtreiberkolonne abgezogen wird, während der Rest des Lösungsmittels mit einem gewissen Aromatengehalt und niedrigerer Temperatur als Seitenstrom aus der Abtreiberkolonne abgezogen wird, wobei dieser Seitenstrom an einer Stelle in die mit Rückfluß betriebene Extraktivdestillationskolonne wiedereingeleitet wird, die 6 bis 10 Böden unterhalb der Eintrittsstelle für den vom Sumpf der Abtreiberkolonne kommenden Lösungsmittelstrom liegt.

2. Verfahren nach Anspruch 1, **dadurch gekennzeichnet,** daß 55 bis 60 Gew.-% der Gesamtlösungsmittelmenge aus dem Sumpf und der Rest als Seitenstrom aus der Abtreiberkolonne abgezogen werden.

3. Verfahren nach den Ansprüchen 1 und 2, **dadurch gekennzeichnet,** daß der aus dem Sumpf der Abtreiberkolonne abgezogene Lösungsmittelteilstrom vor seiner Wiedereinleitung in die Extraktivdestillationskolonne in an sich bekannter Weise gekühlt wird, während bei dem als Seitenstrom aus der Abtreiberkolonne abgezogenen Lösungsmittelteilstrom eine Kühlung vor der Wiedereinleitung in die Extraktivdestillationskolonne nicht vorgesehen ist.

4. Verfahren nach den Ansprüchen 1 bis 3, **dadurch gekennzeichnet,** daß die Extraktivdestillation mit einem Rückflußverhältnis von 1,5 bis 2,5 betrieben wird.

5. Verfahren nach den Ansprüchen 1 bis 4, **dadurch gekennzeichnet,** daß die Extraktivdestillation in einer Kolonne durchgeführt wird, die oberhalb der Lösungsmittelaufgabe einen zusätzlichen Kolonnenanteil für die Abtrennung der Lösungsmittelreste von den gewonnenen Nichtaromaten aufweist.

## Claims

1. Process for separating aromatics from hydrocarbon mixtures of any possible aromatics content by extractive distillation with a selective solvent, in which the non-aromatic constituents of the hydrocarbon mixture used as feed product are distilled off over the top of the extractive distillation column, while the aromatics together with the solvent used are taken off from the bottom of the extractive distillation column and separated from the solvent by distillation in a downstream stripper column, the aromatics being taken off as the top product and the solvent being taken off as the bottom product from the stripper column, whereupon the solvent is recycled to the extractive distillation column, characterised in that only a part of the solvent is taken off at the high temperature, required for complete stripping of the aromatics, from the bottom of the stripper column, while the remainder of the solvent is taken off from the stripper column as a side stream with a certain aromatics content and at a lower temperature, this side stream being recycled to the extractive distillation column, operated with reflux, at a point which is 6 to 10 trays below the inlet point for the solvent stream coming from the bottom of the stripper column.

2. Process according to Claim 1, characterised in that 55 to 60% by weight of the total quantity of solvent is taken off from the bottom and the remainder is taken off as a side stream from the stripper column.

3. Process according to Claims 1 and 2, characterised in that the solvent part stream taken off from the bottom of the stripper column is cooled in a manner known per se before it is recycled to the extractive distillation column while no cooling is provided for the solvent part stream taken off as a side stream from the stripper column before it is recycled to the extractive distillation column.

4. Process according to Claims 1 to 3, characterised in that the extractive distillation is operated at a reflux ratio of from 1.5 to 2.5.

5. Process according to Claims 1 to 4, characterised in that the extractive distillation is carried out in a column which, above the solvent feed, has an additional column section for separating the solvent residues from the non-aromatics produced.

## Revendications

1. Procédé de séparation de produits aromatiques à partir de mélanges d'hydrocarbures à teneur en produits aromatiques quelconque par distillation extractive à l'aide d'un solvant sélectif, lors duquel les constituants non aromatiques du mélange d'hydrocarbures servant de produit de charge sont éliminés par distillation en tête de colonne hors de la colonne de distillation extractive, alors que les produits aromatiques, conjointement au solvant utilisé, sont extraits du bas de colonne de la colonne de distillation extractive et sont séparés du solvant par distillation dans une colonne de strippage qui est placée à la suite, les produits aromatiques étant extraits de la colonne de strippage en tant que produits dé tête de colonne et le solvant en tant que produit de bas de colonne, le solvant étant réintroduit dans la colonne de distillation extractive, **caractérisé en ce que** seulement une partie du solvant ayant la température élevée nécessaire à l'élimination complète des produits aromatiques est extraite du bas de colonne de la colonne de strippage, alors que le reste du solvant ayant une teneur déterminée en produits aromatiques et une température plus faible est extrait de la colonne de strippage en tant que produit de soutirage latéral, ce produit de soutirage latéral étant réintroduit à un point dans la colonne de distillation extractive fonctionnant avec reflux, qui se situe de 6 à 10 plateaux endessous du point d'admission pour le courant de solvant venant du bas de colonne de la colonne de strippage.

2. Procédé selon la revendication 1, **caractérisé en ce que** de 55 à 60 % en poids de la quantité totale de solvant sont extraits en provenance du bas de colonne, et que le reste est extrait de la colonne de strippage en tant que produit de soutirage latéral.

3. Procédé selon les revendications 1 et 2, **caractérisé en ce que** le courant partiel de solvant extrait du bas de colonne de la colonne de strippage avant sa réintroduction dans la colonne de distillation extractive est refroidi d'une manière en soi connue, alors que, pour ce qui est du courant partiel de solvant extrait de la colonne de strippage en tant que produit de soutirage latéral, il n'est pas prévu de refroidissement avant la réintroduction dans la colonne de distillation extractive.

4. Procédé selon les revendications 1 à 3, **caractérisé en ce que** la distillation extractive fonctionne avec un rapport de reflux de 1,5 à 2,5.

5. Procédé selon les revendications 1 à 4, **caractérisé en ce que** la distillation extractive est effectuée dans une colonne qui présente au-dessus du point d'admission de solvant une partie de colonne supplémentaire destinée à la séparation des restes de solvant des produits non aromatiques obtenus.
